# NEW EUROPEAN PATENT SPECIFICATION

(11) **EP 0 540 858 B2**
(45) Date of publication and mention of the opposition decision: **20.02.2008**
(45) Mention of the grant of the patent: 08.08.2001
(21) Application number: 92115587.5
(22) Date of filing: 11.09.1992
(51) Int. Cl.: A61L 29/00

(54) **Inflatable member having elastic expansion with limited range**
In begrenztem Bereich elastisch dehnbarer aufblasbarer Gegenstand
Objet gonflable ayant une expansion élastique limitée

(30) Priority: 12.09.1991 US 758630; 23.07.1992 US 918232
(43) Date of publication of application: 12.05.1993
(60) Divisional application: 99119233.7
(73) Proprietor: ADVANCED CARDIOVASCULAR SYSTEMS, INC., Santa Clara California 95054 (US)
(72) Inventor: Muni, Ketan P., San Jose, California 95136 (US); Bagaoisan, Celso Jacinto, Newark, California 94560 (US); Powers, Gerard F., San Ramon, California 94583 (US); Samson, WilfredJ., Saratoga, California 95070 (US); Taylor, Kevin D., San Jose, California 95148 (US)
(74) Representative: Naylor, Matthew John

(56) References cited:
- EP-A- 0 274 411
- EP-A- 0 349 640
- EP-A- 0 359 489
- EP-A- 0 409 436
- EP-A- 0 456 342
- EP-A2- 0 197 787
- GB-A- 2 008 140
- US-A- 4 490 421
- US-A- 4 638 805
- US-A- 4 711 624
- US-A- 4 748 982
- US-A- 4 932 959
- US-A- 5 021 043
- US-A- 5 035 694
- Yeatman et al, "Angioplasty Balloons, ...", Endovascular Surgery, Chapter 20, pp. 148-155, 1989
- Gray et al: "Marked Diameter Enlargement in Polyolefincopolymer Angioplasty Balloons After First Inflation", Abstract presented at the 1991 AHA, Anaheim California, November 11-14, 1991, Circulation Volume 84, 4. October 1991, p. II-591
- Jain et al: "Effect of Inflation Pressures on Coronary Angioplasty Balloons", American Journal of Cardiology", Vol. 57, pages 26-28, 1986
- Data Sheet Cordis Orion: "Steerable PTCA Balloon Catheter Pressure vs. Diameter Curves", June 1990
- SCIMED Technical Bulletin: "Balloon Creep after Serial Inflations: ...", October 1991

## Description

### BACKGROUND OF THE INVENTION

This invention generally relates to intravascular catheters, such as balloon dilatation catheters used in percutaneous transluminal coronary angioplasty (PTCA).

PTCA is a widely used procedure for the treatment of coronary heart disease. In this procedure, a balloon dilatation catheter is advanced into the patient's coronary artery and the balloon on the catheter is inflated within the stenotic region of the patient's artery to open up the arterial passageway and thereby increase the blood flow therethrough. To facilitate the advancement of the dilatation catheter into the patient's coronary artery, a guiding catheter having a preshaped distal tip is first percutaneously introduced into the cardiovascular system of a patient by the Seldinger technique through the brachial or femoral arteries. The catheter is advanced until the preshaped distal tip of the guiding catheter is disposed within the aorta adjacent the ostium of the desired coronary artery. The guiding catheter is twisted or torqued from the proximal end, which extends out of the patient, to guide the distal tip of the guiding catheter into the ostium. A balloon dilatation catheter may then be advanced through the guiding catheter into the patient's coronary artery until the balloon on the catheter is disposed within the stenotic region of the patient's artery. The balloon is inflated to open up the arterial passageway and increase the blood flow through the artery.

One type of catheter frequently used in PTCA procedures is an over-the-wire type balloon dilatation catheter. Commercially available over-the-wire type dilatation catheters include the SIMPSON ULTRA-LOW PROFILE® , the HARTZLER ACX® , the HARTZLER ACX II® , the PINKERTON .018^{™} and the ACS TEN^{™} balloon dilatation catheters sold by the assignee of the present invention, Advanced Cardiovascular Systems, Inc. (ACS). When using an over-the-wire dilatation catheter, a guidewire is usually inserted into an inner lumen of the dilatation catheter before it is introduced into the patient's vascular system and then both are introduced into and advanced through the guiding catheter to its distal tip which is seated within the ostium. The guidewire is first advanced out the seated distal tip of the guiding catheter into the desired coronary artery until the distal end of the guidewire extends beyond the lesion to be dilatated. The dilatation catheter is then advanced out of the distal tip of the guiding catheter into the patient's coronary artery, over the previously advanced guidewire, until the balloon on the distal extremity of the dilatation catheter is properly positioned across the lesion to be dilatated. Once properly positioned across the stenosis, the balloon is inflated one or more times to a predetermined size with radiopaque liquid at relatively high pressures (*e.g.*, generally 4-12 atmospheres) to dilate the stenosed region of a diseased artery. After the inflations, the balloon is finally deflated so that the dilatation catheter can be removed from the dilated stenosis to resume blood flow.

Fixed-wire type dilatation catheter systems are also utilized very frequently in PTCA procedures. This type of dilatation catheter has guidewire or guiding member secured within the catheter and it provides a low profile, *i.e.* small transverse dimensions, because there is no inner tubular member which is characteristic of commercially available over-the-wire dilatation catheters. Commercially available fixed-wire dilatation catheters include the HARTZLER EXCEL® , the HARTZLER LPS® and the SLALOM^{™} dilatation catheters sold by ACS.

Another type of dilatation catheter, the rapid exchange type catheter, was introduced by ACS under the trademark ACS RX® Coronary Dilatation Catheter. It is described and claimed in U.S. Patent 5,040,548 (Yock), U.S. Patent 5,061,273 (Yock), and U.S. Patent 4,748,982 (Horzewski et al.) which are incorporated herein by reference. This dilatation catheter has a short guidewire receiving sleeve or inner lumen extending through a distal portion of the catheter. The sleeve or inner lumen extends proximally from a first guidewire port in the distal end of the catheter to a second guidewire port in the catheter spaced proximally from the inflatable member of the catheter. A slit may be provided in the wall of the catheter body which extends distally from the second guidewire port, preferably to a location proximal to the proximal end of the inflatable balloon. The structure of the catheter allows for the rapid exchange of the catheter without the need for an exchange wire or adding a guidewire extension to the proximal end of the guidewire. This catheter has been widely praised by the medical profession and it has met with much success in the marketplace because of the advantages of its unique design.

The perfusion type dilatation catheter was another type of dilatation catheter introduced into the marketplace by ACS. This catheter, which can take the form of an over-the-wire catheter or a rapid exchange type catheter, has one or more perfusion ports proximal to the dilatation balloon in fluid communication with an guidewire receiving inner lumen extending to the distal end of the catheter. One or more perfusion ports are preferably provided in the catheter distal to the balloon which are also in fluid communication with the inner lumen extending to the distal end of the catheter. When the balloon of this dilatation catheter is inflated to dilate a stenosis, oxygenated blood in the artery or the aorta or both, depending upon the location of the dilatation catheter within the coronary anatomy, is forced to pass through the proximal perfusion ports, through the inner lumen of the catheter and out the distal perfusion ports. This provides oxygenated blood downstream from the inflated balloon to thereby prevent or minimize ischemic conditions in tissue distal to the catheter. The perfusion of blood distal to the inflated balloon allows for long term dilatations, e.g. 30 minutes or even several hours or more. This catheter has likewise been highly praised by the medical profession and has met with much commercial success. Commercially available perfusion type dilatation catheters include the STACK PERFUSION^{™} and the ACS RX PERFUSION^{™} Dilatation Catheters which are sold by ACS.

The balloons for prior dilatation catheters utilized in angioplasty procedures generally have been formed of relatively inelastic polymeric materials such as polyvinyl chloride, polyethylene, polyethylene terephthalate and polyolefinic ionomers. Nylon has been mentioned in the literature as an alternative inelastic material from which dilatation balloons can be made, but there has not been much commercial use of this material. The aforementioned prior art balloons are characteristically relatively inelastic so that upon inflation with inflation liquid there is relatively little expansion of the balloon with increased internal pressures, even at very elevated levels. However, when the prior art balloons were deflated, the inelastic balloon material did not shrink or contract, so as a result, the deflated profiles of the prior art balloons were relatively large. In an effort to reduce the deflated profiles of the prior art balloons made formed polyethylene, polyvinyl chloride and polyolefinic ionomers. very frequently they would be heat formed so as to wrap around inner members extending through the interior of the balloons. However, balloons formed of polyethylene terephthalate were not readily heat formed, with the result that, when a vacuum was pulled on the balloon, wings were formed which extend outwardly presenting a relatively large profile.

What has been needed and heretofore unavailable is a thin walled inflatable member for intravascular catheters which upon inflation exhibits a controlled elastic expansion but which does not expand significantly beyond a particular pressure level. The present invention satisfies these and other needs.

### SUMMARY OF THE INVENTION

This invention is directed to an inflatable member such as a balloon which exhibits upon inflation a substantial elastic expansion within a first pressure range and which is considerably less compliant at pressures beyond the first pressure range. Upon deflation, the inflatable member contracts by elastic recoil to a diameter much smaller than the inflated diameter.

Disclosed herein is an inflatable member having an expandable wall portion which at internal pressures within a first pressure range exhibits substantial elastic expansion and within a second pressure range, higher than the first pressure range, exhibits relatively little expansion.

The inflatable member of the invention is a tubular member which when inflated exhibits a relatively high rate of elastic expansion within a first range of internal pressures and a relatively low rate of expansion, i.e. is much less compliant, at pressures within a second range of internal pressures higher than the first range. In one presently preferred embodiment, during the initial stage of inflation, when the internal pressures are below the first pressure range, the inflatable member or balloon is relatively noncompliant and experiences relatively little expansion, but when the internal pressures reach the first pressure range, the inflatable member expands elastically at a relatively high rate until the pressure enters a second pressure range at which point the inflatable member becomes relatively noncompliant and the expansion rate thereof is quite low. The expansion at failure is usually less than 25% and preferably less than 10% of the maximum inflated diameter at the end of the elastic expansion. Upon deflation of the inflatable member, it contracts to a diameter much smaller than the inflated diameter by means of elastic recoil.

In the deflated condition the inflatable member of the invention preferably has outer dimensions which are essentially the same as or not much larger than adjacent portions of the catheter shaft in order to present a relatively smooth outer surface which greatly facilitates the insertion and advancement of the inflatable member through the vascular system of a patient and through stenotic region of the patient's artery. When subjected to a vacuum, the inflatable member forms very small wings or essentially no wings at all which helps the passage of the inflatable member through the patient's blood vessels and through stenoses.

The inflatable member of the invention may be formed of heat shrinkable thermoplastic material, particularly a radiation cross-linked polymer material, which has been thermally treated at a temperature of not more than about 50° C above and not more than about 50° C below the crystalline melting point of the polymer to provide the requisite expansion of the present invention. In one presently preferred embodiment the inflatable member is formed of a polyolefinic ionomer such as those sold under the trademark Surlyn® by E.I. duPont, deNemours & Co. and particularly Surlyn® 8020/IBE which is a sodium cation ionomer. Other suitable ionomers include sodium ionomers 8920 and 8940, zinc ionomer 9020 and lithium ionomers 7930 and 7940 which are all sold under the trademark Surlyn® . An inflatable member or balloon formed of this material and treated in the prescribed manner has at body temperature a radial expansion which is generally elastic and predictable at pressures within a particular first range of pressures and which is considerably less compliant at pressures above the range of pressures. In another presently preferred embodiment, the cross-linked ionomer is a zinc ionomer sold under the designation F1855 by E.I. duPont, deNemours & Co. which is a lower molecular weight variant of the Surlyn® 9020 zinc ionomer mentioned above. Other heat shrinkable polymers include high density and low density polyethylene and mixtures thereof and ethylene vinyl acetate such as EVAC® sold by duPont, deNemours & Co. may also be treated in the above manner to provide such properties. However, most thermoplastic polymers other than the ionomers, usually do not exhibit the requisite expansion characteristics at the pressure levels normally employed in angioplasty procedures, i.e. they exhibit these characteristics at much higher pressures. However, by heating the inflatable member formed of non-ionomer polymers while it is inflated, the pressure levels at which the polymer will exhibit the requisite expansion characteristics will be lowered considerably.

In one embodiment of the invention an inflatable member which has the desired elastic expansion at body temperature (37° C) is provided on the distal portion of a catheter adapted for intravascular procedures such as angioplasty. The catheter has an elongated shaft with proximal and distal extremities and an inner lumen extending therein for directing inflation fluid therethrough to the interior of the inflatable member. The inflatable member may be formed of the same material as the entire catheter shaft or only a distal portion of the shaft or it may be formed of the same or different materials and secured by suitable means such as adhesive, heat bonding or heat shrinking to the distal end of the shaft. An adapter is provided on the proximal end of the elongated shaft to direct inflation fluid through the inner lumen of the catheter shaft to the interior of the inflatable member.

The inflatable member of the invention can be used in essentially all dilatation catheters for angioplasty procedures including those described in the Background of the Invention. By heat treating only a portion of the inflatable member, *e*.*g*. along one side, only one side of the inflatable member will inflate. This can be advantageously used with eccentric lesions within the patient's artery. The inflatable member of the invention may also be used in other types of catheters. For example, they may be employed with atherectomy devices such as described in U.S. Reissue Patent 33,569, which is incorporated herein by reference, to position the cutting head at a desired position within the blood vessel or they can be employed to adjust the pressure of the cutting head against the atherosclerotic mass to force more or less of the mass within the cutting chamber of the cutting head. The inflatable member of the invention can also be used in catheters such as described in U.S. Patent 4,932,959 (Songer), incorporated herein by reference, which have means to releasably secure a guidewire within an inner lumen within the interior of the balloon without inflating the balloon to provide increased pushability to the catheter to facilitate crossing tight or completely occluded lesions.

The relatively noncompliant nature of the balloon material at inflation pressures greater than the inflation pressures within the first pressure range provides a great degree of ensurance that the inflatable member does not over inflate within the patient's vasculature which could cause damage to the arterial wall or other body lumen in which the inflatable member is disposed. Moreover, to the extent that the balloon is inflated to pressures greater than the first pressure range, the small amount of balloon expansion which does occur can be controlled by the physician by observing the pressure of the inflation fluid within the catheter. If the physician finds that the conditions within the blood vessel require an inflatable member or balloon of a slightly greater diameter than the diameter originally contemplated, all the physician needs to do is to adjust the pressure of the inflation fluid within the interior of the balloon to a predetermined level to safely provide an inflated balloon diameter of the desired size.

For inflatable members formed of many presently available polymer systems, the first pressure range, wherein the expansion is in the elastic mode, can be very high and in some instances can be too high for the intravascular and intraluminal uses contemplated herein. It has been found that the expansion of the inflatable member can be initiated at much lower pressures by heat treating and preexpanding the inflatable member at the heat treat temperature, cooling the preexpanded member and then heat shrinking it. However, in this case the maximum pressure at which the expandable member elastically expands remains essentially the same, i.e. the rate of elastic expansion of the inflatable member decreases but the maximum pressure within the elastic pressure range does not significantly change. In this embodiment of the invention, the initial diameter of the deflated inflatable member is larger than the diameter of the inflatable member which has not been preexpanded and heat shrunk. However, upon applying a vacuum to the interior of the inflatable member in accordance with the present invention, it has been found to readily form small wings which tend to wrap around any inner tubular member to reduce the deflated profile of the catheter. The relatively small wings allow the inflatable member to expand upon inflation without applying significant shear stress to the stenotic region. There is much evidence demonstrating that high shear stress on the lesion can cause dissections which can interfere with blood flow through the arterial passageway and that high shear stress can develop an arterial lining on which restenosis is rapid.

In yet another embodiment of the invention, means are provided to heat the inflatable member after it has been inserted into a patient's vasculature or other body lumen to reduce the pressure required to inflate the inflatable member to an operable size. Suitable systems for heating the inflatable member by radio frequency energy are disclosed in copending applications Serial No. 07/351,777, filed May 15, 1989 and Serial No. 07/521,337, filed May 9, 1990 which are incorporated herein.

The inflatable members of the invention may also be employed in dilatation catheters used in other body lumens such as the catheter described in copending application Serial No. 07/483,397, filed February 14, 1990 which is adapted to dilate a prostatic urethra subject to hyperplasia.

Catheters having inflatable members in accordance with the invention may also be used to deliver expandable tubular elements mounted on the exterior of the inflatable member. Examples of such expandable tubular elements include stents and tubular elements which accept drug or therapeutic fluid and which expel drugs or therapeutic fluids upon the expansion of the inflatable member, *i.e*. the wall of the tubular element thins upon expansion thereby driving out the fluid.

These and other advantages of the invention will become more apparent from the following detailed description thereof when taken in conjunction with the accompanying exemplary drawings.

### BRIEF DESCRIPTION OF THE DRAWINGS

FIG. 1 is an elevational view partially in section of a dilatation catheter embodying features of the invention.

FIG. 2 is a transverse cross-sectional view of the catheter shown in FIG. 1 taken along the lines 2-2.

FIG. 3 is a transverse cross-sectional view of the catheter shown in FIG. 1 taken along the lines 3-3.

FIG. 4 is an elevational view of the distal portion of the catheter shown in FIG. 1 with the inflatable section in an inflated condition.

FIG. 5 is a graphical representation of the relationship of the outer diameters of inflatable members of the invention with respect to the internal pressure.

FIG. 6 is a longitudinal cross-sectional view of the distal portion of a dilatation catheter embodying features of the invention having means to releasably secure a guidewire within the catheter.

FIG. 7 is a longitudinal cross-sectional view of a distal portion of a dilatation catheter as shown in FIG. 6 wherein the means to releasably secure a guidewire within the catheter is engaged with the guidewire.

FIG. 8 is a longitudinal cross-sectional view of a distal portion of a dilatation catheter embodying features of the invention having means to heat the inflatable section.

FIG. 9 is a transverse cross-sectional view of the distal portion of the dilatation catheter shown in FIG. 8 taken along the lines 9-9.

FIG. 10 is a transverse cross-sectional view of the distal portion of the dilatation catheter shown in FIG. 8 taken along the lines 10-10.

FIG. 11 is a longitudinal view of a distal portion of a dilatation catheter embodying features of the invention having an expandable tubular element which is capable of absorbing drugs or other therapeutic fluids mounted on the exterior of the inflatable section of the catheter.

FIG. 12 is a transverse cross-sectional view of the embodiment shown in FIG. 11 taken along the lines 12-12.

FIG. 13 is a longitudinal cross-sectional view of the embodiment in FIG. 11 with the inflatable section in an inflated condition.

FIG. 14 is a longitudinal cross-sectional view of the distal portion of a steerable, fixed-wire dilatation catheter having an inflatable section which embodies features of the invention.

### DETAILED DESCRIPTION OF THE INVENTION

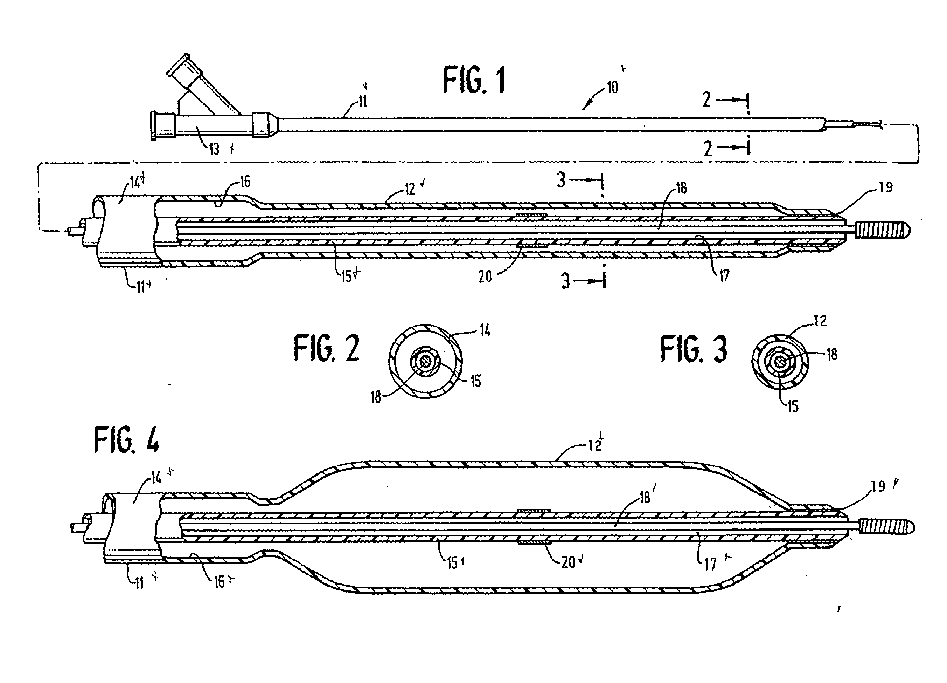

Reference is made to FIGS. 1-3 which illustrate a dilatation catheter 10 embodying features of the invention. The dilatation catheter 10 generally includes a catheter shaft 11 with an inflatable tubular section 12 on the distal extremity of the catheter and an adapter 13 on the proximal end thereof and. The catheter shaft 11 has an outer tubular member 14 which is provided with the inflatable tubular section 12 and an inner tubular member 15 which is disposed within the outer tubular member 14 and defines with the outer tubular member an annular inflation lumen 16 which is adapted to direct inflation fluid from the proximal end of the catheter 10 to the interior of the inflatable section 12 of the catheter.

The inner tubular member 15 has an inner lumen 17 extending from the proximal to the distal end thereof which is adapted to receive a guidewire 18. The distal end of the inflatable portion 12 of the outer tubular member 14 is bonded by a suitable adhesive 19, e.g. a cyanoacrylate based adhesive such as Loctite® 414, to the distal end of the inner tubular member 15 to thereby seal the interior of the inflatable section 12 to prevent the loss of inflation fluid. While not shown in the drawings, a means such as one or more small passageways to vent air from the interior of the inflatable portion may be provided, such as described in U.S. Patent 4,638,805 (Powell) and U.S. Patent 4,821,722 (Miller et al.) which are incorporated herein by reference. A radiopaque marker 20 is provided about the portion of the inner tubular member 15 which extends through the interior of the inflatable section 12. The marker 20 is located at the midpoint of the inflatable section 12 to facilitate fluoroscopic observation thereof when the catheter is disposed within a patient's vascular system or other body lumen.

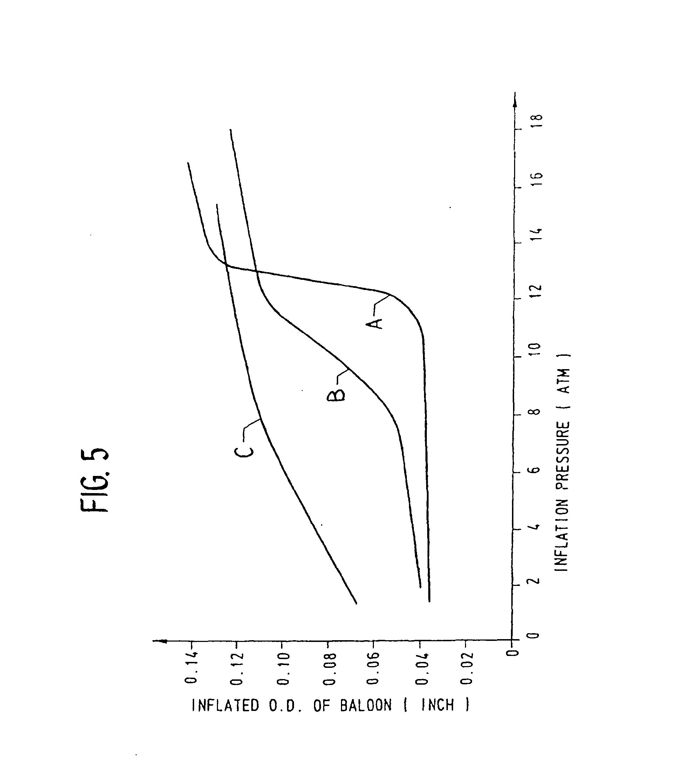

The expansion of the inflatable section 12 of the outer tubular member 14 upon the introduction of inflation fluid within the interior thereof, as described in the example below, is illustrated in FIG. 5.

The following example is given to further illustrate features of the invention. An outer tubular member 14 for a dilatation catheter 10 was prepared having a structure essentially as shown in FIGS. 1-3 and made of Surlyn® (8020 grade), a sodium ionomer supplied by the E.I. duPont, deNemours & Company. The outer tubular member 14 has an OD of about .093 cm (0.037 inch) and an ID of about .063 cm (0.025 inch), *i.e.* a wall thickness of about .015 cm (0.006 inch) over essentially its entire length. The outer tubular member 14 has irradiated at a level of about 45 to 55 mrads in order to cross-link essentially the entire polymeric tube. The distal portion of the polymerized tubular member which was to become the inflatable portion 12 of the catheter 10 was subjected to a thermal treatment at about 250° F for a period of about 20 seconds while applying tension in the longitudinal direction in order to develop a significant level of longitudinal orientation in the inflatable portion. After the thermally treated inflatable section 12 of the tubular member had cooled to room temperature, the dilatation catheter 10 was assembled. The interior of the inflatable section 12 of the catheter was subjected to increasing internal pressures ranging from atmospheric to a burst pressure of about 20 atmospheres and the outer diameter of the inflatable member was determined at the various internal pressures. The relationship between the outer diameter of the inflatable section 12 and the fluid pressure within the inflatable tubular section is shown as curve A in FIG. 5. As is evident, at pressures from atmospheric to about 12 atmospheres, the change in the outer diameter of the inflatable section was negligible, *i.e.* from .093 cm (0.037 in) to about .10 cm (0.04 inch) indicating that the material had little compliance within this pressure range. At pressures from about 12 to about 14 atmospheres the material exhibited a substantial (three-fold) elastic expansion from .091 cm (0.036 in) to about .292cm (0.115 inch). At internal pressures beyond the range of 12 to 14 atmospheres, the increase in the outer diameter of the inflatable section 12 was very small, *i.e*. less than about 10% of the maximum diameter within the elastic expansion range, indicating noncompliance. Even though the expansion was very small, , it was essentially linear with respect to the interior pressure within the inflatable section and was therefore predictable.

When the inflatable section 12 is inflated to a larger diameter, frequently a differential develops between the length of the inflated inflatable section and the underlying portion of the inner tubular member 15, *i.e*. the outer tubular member 14 shrinks in the longitudinal direction, causing the inflated inflatable section to become deformed. To minimize or eliminate the differential, the inflatable section of the outer tubular member 14 is first formed into a balloon by subjecting this section to an internal pressure at elevated temperatures, *e.g.* from about 121°C (250° F), in a conventional manner and cooling the balloon while it is inflated, *e*.*g*. to room temperature, to impart heat shrinking characteristics. After cooling, the inflated inflatable portion 12 is deflated. The outer tubular member 14 is then assembled with the inner tubular member 15 and then the treated inflatable section 12 is heat shrunk onto an inner member such as the inner tubular member 15 or a mandrel (not shown) thereby eliminating or minimizing differential elongations upon the inflation of the inflatable section. Heat shrinking is effected by heating to a temperature of about 50° to about 80° C, preferably about 55° to about 65° C for at least about 5 seconds and generally not longer than about 1 hour. The heating for first forming the balloon prior to the heat shrinking thereof can be heat treatment to provide the expansion characteristics of the invention which is generally within the range of 50° C above or below the crystalline melting point of the polymer.

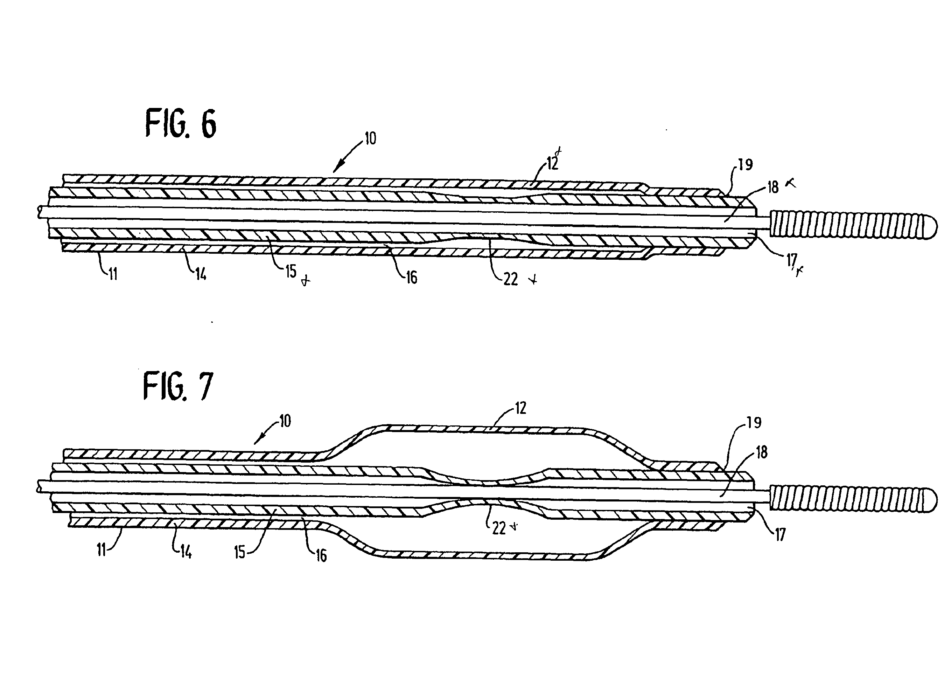

FIGS. 6 and 7 illustrate a modified embodiment of the invention having means to releasably secure a guidewire 18 within the catheter 10 so that the catheter and the guidewire can be advanced together as a unit through an artery and a stenosis within the artery as described in U.S. Patent 4,932,959 (Horzewski et al.) which has been incorporated herein. In the embodiment shown in FIGS 6 and 7, the inner tubular member 15 is provided with a thin wall section 22 which is designed to collapse onto a guidewire 18 disposed within the inner lumen 17 at a pressure less than the pressure range in which the inflatable member 12 expands elastically. In this manner, the annular inflation lumen 16 of the catheter may be subjected to a first pressure much less than the pressure range effecting elastic expansion to cause the thin wall section 22 to collapse about the guidewire 18 thereby releasably securing it within the catheter. The combined catheter and guidewire assembly has much better pushability than either the catheter or the guidewire alone so the assembly can be more easily advanced through a tight stenosis. Once the inflatable portion 12 of the dilatation catheter 10 is disposed across the stenosis, the pressure of the inflation fluid within the catheter may then be increased to a level above the threshold level to inflate the inflatable section 12 to the desired size to dilate the stenosis. After the dilatation, the pressure of the inflation fluid may be reduced to a level which allows the inflated section 13 to return to its original size. If no further dilatations are to be done, the pressure may be reduced to even lower levels to allow the thin wall section 22 of the inner tubular member 15 to return to its original position, thereby releasing the guidewire 18 therein.

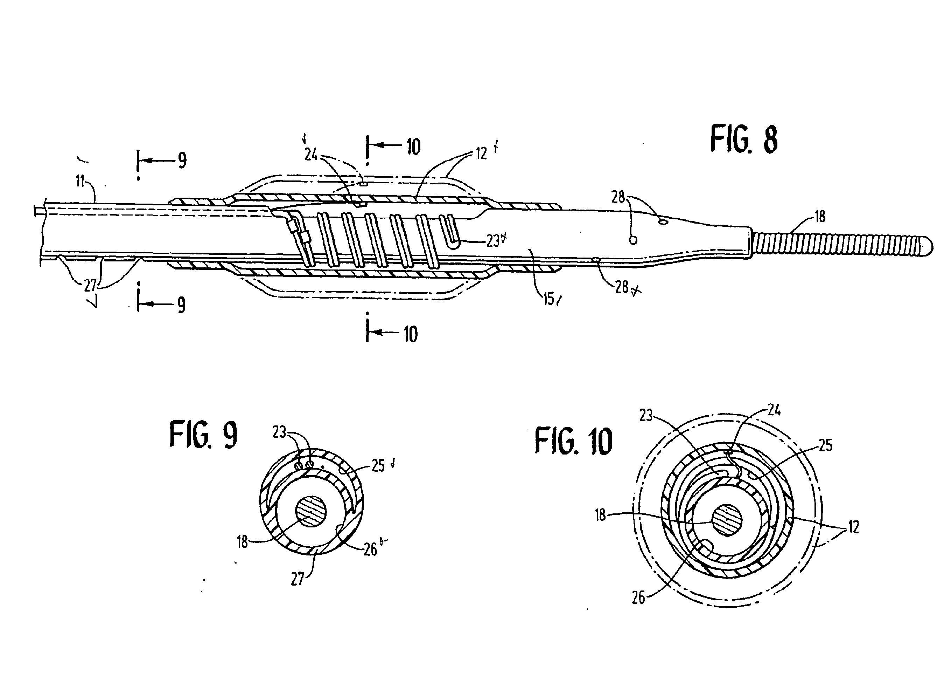

FIGS. 8-10 illustrate yet another embodiment of the invention wherein the inflatable member 12 is heated while it is being inflated within the patient in order to reduce the pressure range in which substantial expansion of the inflatable member occurs in the elastic mode. In this embodiment, a heater coil 23, which is electrically connected to an electrical power source (not shown), is disposed about the inner tubular member 15 which extends through the interior of the inflatable section 12. A thermocouple 24 may be provided to sense the temperature of the inflatable section 12 or the inflation fluid therein so that the control means (not shown) may compare the temperature sensed with a desired temperature limit and adjust the electrical power from the source accordingly to control the temperature as desired. The pressure and temperature relationship with respect to the inflated diameter of the inflatable section are readily determined before the insertion of the catheter within a patient's vasculature so that the desired inflated diameter of the inflatable section can be obtained by the physician by noting the pressure and adjusting the temperature of the inflatable wall portion of the balloon or vice versa. Generally, a rise in the temperature of the wall of the inflatable section will lower the pressure range wherein there is a substantial elastic expansion of the inflatable section as shown in FIG. 5 and reduce somewhat the rate of pressure increase. Once the inflatable member has been inflated above the pressure range for elastic expansion, the temperature of the inflatable member can be allowed to return to body temperature and the angioplasty or other procedure can be completed in a conventional fashion. The catheter shaft 11 of this embodiment differs somewhat from that shown in the prior embodiments in that it has two inner lumens extending side-by-side therein, a first or inflation lumen 25 which is crescent shaped in transverse cross-section and a second or guidewire receiving lumen 26 which is circular in cross-section as shown in FIG. 9. The inflation section 13 is shown in the inflated condition in phantom in FIGS. 8 and 10. In this embodiment proximal and distal perfusion ports 27 and 28 respectively are provided so, that upon inflation of the inflatable section 13 to dilate a stenosis, oxygenated blood will flow through the proximal perfusion ports 27 into the inner lumen 17 and out the distal perfusion ports to reduce the possibility of ischemic conditions developing in tissue distal to the catheter.

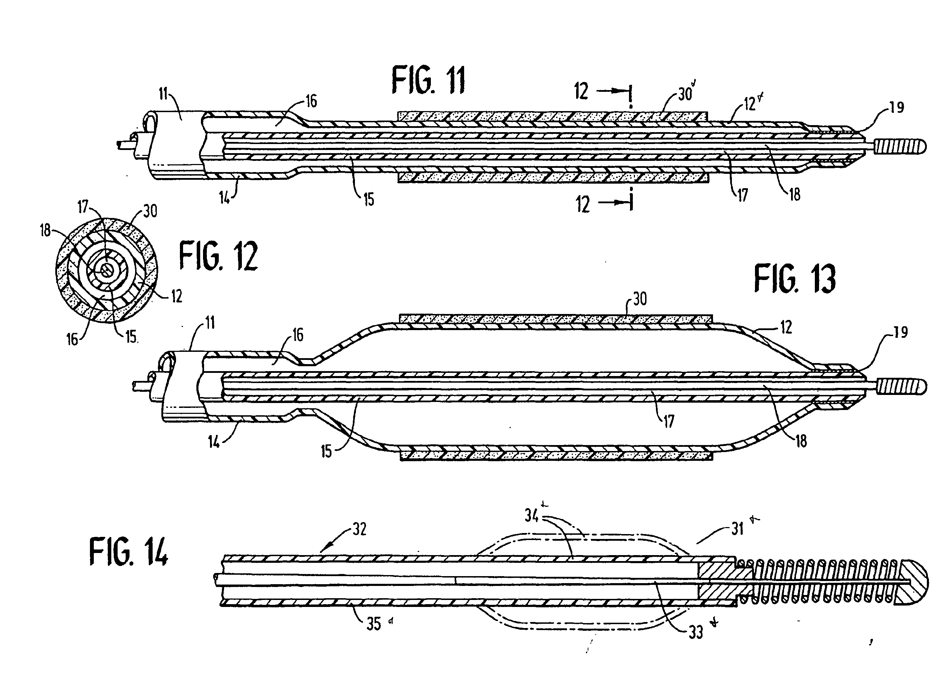

FIGS 11-13 illustrate another embodiment of the invention wherein the catheter 10 is adapted to deliver drugs to a desired location within a patient's body lumen, such as a blood vessel. In the embodiment shown a tubular element 30 is capable of absorbing liquid drugs or therapeutic fluids is disposed about the inflatable section 12 of the invention. Inflation of the inflatable section 12 increases the diameter of the tubular element 30, compressing the wall thereof and driving out fluid absorbed therein to deliver the drug or therapeutic fluid to the desired location within the patient's body lumen. In an alternate embodiment (not shown) a second inflatable member having a plurality of small apertures through the wall thereof is disposed about the inflatable section 12 with liquid drugs or therapeutic fluids disposed between the inflatable section 12 and the second inflatable member so that inflation of the inflatable section will drive the liquid through the apertures in the outer second inflatable member. The number and size of the apertures in the wall of the outer balloon wall are determined for the most part by the nature of the therapeutic fluid, e.g. viscosity and the like, and the amount of fluid to be delivered and the rate at which it is to be delivered.

A steerable, fixed-wire dilatation catheter 31 is depicted in FIG. 14 which has a tubular shaft 32 having a proximal portion (not shown) formed of stainless steel or superelastic Nitinol hypotubing with a guiding member 33 secured by its proximal end to the distal portion of the hypotubing (not shown). An inflatable member 34 embodying features of the invention is disposed about the guiding member 33 with the distal end of the inflatable section 34 being sealingly secured about the portion of the guiding member 33 which extends therethrough. The proximal portion of the balloon is provided with an elongated skirt 35 with a proximal end (not shown) sealed about the distal end of the hypotubing.

A presently preferred embodiment of the invention, as shown in FIGS. 1-4, is a dilatation catheter for PTCA wherein the outer tubular member 14 has an outer diameter of about .0508cm (0.02inch) to about .1016 cm (0.04 inch) (typically about .094 cm (0.037 inch), an inner diameter of about .038cm (0.015 inch) to about .089 cm (0.035 inch) (typically about .076 cm (0.03 inch). The wall thickness of the outer tubular member 14 can vary from about .005cm (0.002 inch) to about 2.03cm (0.008 inch) (typically about .0076 cm (0.003 inch)). The distal inflatable section 12 of the outer tubular member 14 may have an outer diameter of about .0635 cm (0.025 inch) to about .076 cm (0.030 inch), typically about .068 cm (0.027 inch) and an inner diameter of about .05 cm (0.020 inch) to about .0635 cm (0.025 inch), typically about .058 cm (0.023 inch). The wall thickness will vary depending upon the burst pressure desired but generally will range from about .0025 cm (0.001 inch) to about .0076 cm (0.003 inch). The inner tubular member 15 has an outer diameter of about .03 cm (0.012 inch) to about .041 cm (0.016 inch), typically about .035 cm (0.014 inch). The overall length of the catheter 10 may range from about 100 to about 150 cm but is typically about 135 cm. The length of the inflatable section 12 may range from about 3 to about 30 cm, but typically is about 10 cm. The dimensions of the other embodiments will be similar.

The following is an example of making another presently preferred embodiment of the invention wherein the inflatable member is formed of a zinc polyoleophilic ionomer. In this embodiment pellets of a zinc olefinic ionomer identified as F1855 (a low molecular weight variant of 9020 Surlyn® from E.I. duPont) are extruded at a temperature between about 177°C (350°F) to about 232°C (450°F) into tubular stock. Upon exiting from the extrusion die, the tubular stock is quenched in a trough of cool water, preferably about 7.2-12.7°C(45-55°F) in order to form a highly amorphous tubular product. The cooled tubular product is stabilized at about 4.4-26.6°C (40 to 80°F), typically about 15.5°C(60°F) for about 2 to about 6 hours, typically about 4 hours. The stabilized tubular product is then irradiated at about 45 to about 75 Mrads, preferably about 55 to about 65 Mrads to cross-link the product. The portion of the tubular product which is to be formed into the inflatable member is then heat treated at a temperature of about 107°C (225°F) to about 121°C (250°F) and subjected to an internal pressure of about 345x10³ Pa (50 psi) to about 586x10³ Pa (85 psi) preferably about 413x10³ Pa (60 psi) to about 517x10³ Pa (75 psi), to expand or blow the heat treated portion of the tubular member into a balloon. The balloon is blown slightly larger than the desired inflated size, *e.g.* up to 3.1 mm if an inflated diameter of 3.0 mm is desired, and then it is assembled with other components into a catheter. The balloon is heat treated at about 55°C to about 65°C for about 10 to about 30 minutes to heat shrink the balloon to a diameter the same or slightly larger than its original diameter. Preferably, a heat shrinkable sheath is placed about the balloon during the heat treatment so that small wings are formed. The curve B in Fig. 5 illustrates the outer inflated diameter of a typical inflatable member at various interior pressures. The starting point of curve B, as shown in the drawing, is after the inflatable member has been sufficiently filled with inflation liquid to form a relatively wrinkle free inflatable member. In the pressure range of about 8 atmospheres and extending to about 12 atmospheres, the expansion rate of the inflatable member is relatively high and the expansion mode is elastic. At pressures beyond about 12 atmospheres the expansion of the inflatable member is relatively low, *i.e*. it is relatively noncompliant. Upon deflation of the inflatable member, the diameter of the inflatable member follows the expansion curve shown in the drawing to essentially the starting point with little or no permanent deformation.

In another example, the same extruded and irradiated tubular product described above, which is formed of zinc olefinic ionomer, was treated by heat treating at about 107° C to about 121° C, but was not inflated at the elevated temperature nor heat shrunk as in the prior example. Curve C in Fig. 5 illustrates a typical relationship between the internal fluid pressure and the outer balloon diameter of inflatable members or balloons which have been formed in this manner.

In yet another example, an olefinic ionomer heated, extruded, cooled and irradiated as above may be subjected to a temperature of about 230° C to 250° C and an inflation pressure of about 345 X 10³ Pa to 586 X 10³ Pa to form an inflated balloon. Then the balloon may be cooled, and the diameter 'of the expanded balloon reduced somewhat by heat shrinking it at a temperature of about 50° C to 75° C for about 5 to 60 minutes.

While the graphical relationship depicted by curves A, B and C may at first glance seem disparate, these curves demonstrate the initial elastic expansion within a first pressure range and the relatively little expansion at pressures above the first pressure range. Upon deflation, the outer diameters of the inflatable members follow the same relationship, exhibiting elastic recoil.

A variety of modifications can be made to the present invention. For example, with the aforementioned preferred embodiment only the distal portion of the outer tubular member 14 that was to form the inflatable section 12 was subjected to the heat treatment. If desired, the entire outer tubular member 14 can be given a thermal treatment but the exterior of the non-inflatable section may be provided with an inelastic jacket or coating so that only the inflatable section 12 inflates when subjected to internal pressure. Other modifications include forming the inflatable section of an outer tubular member in accordance with the' invention and secure the inflatable section to a catheter shaft of different material or the same material with differing properties. In some instances it may be desirable to inflate the inflatable section before the catheter is introduced into the vascular system of the patient in order to reduce the internal pressure required for the initial expansion of the inflatable section. This preexpansion also decreases the rate of increase of the expansion, but does not substantially change the range of pressure in which the elastic expansion occurs. A wide variety of other modifications and improvements can be made to the invention without departing from the scope thereof.

## Claims

1. An elongated catheter (10) **characterised in that** the catheter is an elongated intravascular balloon catheter (10, 31) comprising:
a) an elongated catheter shaft (11,32) having proximal and distal ends, a port in the distal end, a guidewire lumen (7, 26) extending to the port in the distal end to facilitate advancement of the catheter (10) over a guidewire (18) within a patient's vasculature, and an inflation lumen (25, 16) extending therein to a location spaced proximally from the distal end;
b) a balloon (12, 34) on a distal portion of the catheter shaft (11, 32) having an interior in fluid communication with the inflation lumen (25, 16) of the catheter shaft (11, 32) and exhibiting an elastic expansion rate upon inflation within a first pressure range above 8 atmospheres providing a substantial elastic expansion and exhibiting a smaller expansion rate and very little expansion upon inflation within a second pressure range, higher than the first pressure range; and
c) means to direct inflation fluid to the interior of the balloon (12, 34) through the inflation lumen.

2. The catheter (10) of claim 1 further **characterized in that** the catheter shaft (11) has an inner tubular member (15) with the guidewire receiving inner lumen (17, 26) extending therein which extends through the interior of the balloon (12).

3. The catheter (10) of claim 2 further **characterized in that** the inner tubular member (15) has a portion (22) thereof collapsible at a pressure less than the first pressure range.

4. The catheter (10, 31) of any one of claims 1 through 3 further **characterized in that** the balloon (12, 34) is formed of radiation cross-linked polymeric material or heat shrinkable polymeric material.

5. The catheter (10, 31) of claim 4 further **characterized in that** the radiation cross-linked polymeric material is a polyolefinic ionomer selected from the group consisting of sodium, zinc and lithium ionomers.

6. The catheter (10, 31) of any one of claims 1 through 5 further **characterized in that** the balloon (12, 34) exhibits elastic recoil upon deflation.

7. The catheter (10, 31) of any one of claims 1 through 6 further **characterized in that** the balloon (12, 34) is inflated at the thermal treatment temperature, cooled and then heat shrunk.

8. The catheter (10, 31) of any one of the preceding claims further **characterized in that** the deflated transverse dimensions of the balloon (12, 34) are not more than about 10% greater than the transverse dimension of the adjacent catheter shaft (11, 32).

9. The catheter (10, 31) of any one of the preceding claims including means to heat (23) the balloon (12, 34) while it is disposed within a patient's body to lower the first pressure range.

10. The catheter (10) of any one of claims 1 through 9 further **characterized by** the guidewire (18) extending out of the distal end of the balloon (12) with the distal end of the balloon (12) sealably secured about the guidewire (18).

11. A method of making a balloon **characterized by** the steps of:
a) extruding a tubular product formed of an olefinic ionomer at an elevated temperature;
b) cooling the extruded tubular product after its extrusion to obtain a relatively amorphous structure therein;
c) irradiating at least a portion of the amorphous tubular product which will form a balloon; and
d) heat treating the portion of the tubular product which will form the balloon at a temperature between about 50°C above and about 50°C below the crystalline melting temperature.

12. The method of claim 11 further **characterized by** the steps of:
expanding the irradiated portion of the tubular product to a first outer diameter by injecting inflation fluid into the interior of the irradiated portion at an elevated temperature to cause the expansion of the irradiated portion;
cooling the expanded portion of the tubular product;
heat shrinking the expanded portion of the tubular product to a second outer diameter much smaller than the first outer diameter.

13. The method of claim 11 or 12 further **characterized in that** the extruded tubular product is stabilized at a temperature between about 4.4°C (40°F) to about 26.6°C (80°F) for about 2 to about 6 hours before the irradiation thereof.

14. The method of claim 13 further **characterized in that** the extruded tubular product is quenched upon exiting from the extruding operation in a bath at a temperature of about 4°C (40°F) to about 15°C (60°F).

15. The method of claim 11, 12, 13 or 14 further **characterized in that** the extruded tubular product is irradiated with about 50 to about 70 Mrads of gamma radiation.

16. The method of claim 11, 12, 13, 14 or 15 further **characterized in that** the irradiated portion of the extruded tubular product is subjected to a temperature of about 107°C (225°F) to about 121°C (250°F) and an inflation pressure of about 345 x 10³ Pa (50psi) to about 586 x 10³ Pa (85 psi) to form into an inflated balloon (12, 34).

17. The method of claim 16 further **characterized in that** the inflated balloon (12, 34) is cooled and then subjected to a temperature of about 50°C to about 75°C for about 5 to about 60 minutes to heat shrink the expended balloon (12, 34) from a first diameter to a smaller second diameter.

18. The method of any one of claims 11 through 17 wherein said balloon (12, 34) is formed of one or more oleophilic polymers selected from the group consisting of zinc and sodium ionomers.

19. The method of claim 18 further **characterized in that** the balloon is formed of an olefinic ionomer selected from the group consisting of sodium, zinc and lithium olefinic ionomers.

## Patentansprüche

1. Elongierter Katheter (10), **dadurch gekennzeichnet, dass** es sich bei dem Katheter um einen elongierten intravaskulären Ballonkatheter (10,31) handelt, der folgendes umfasst:
a) einen elongierten Katheterschaft (11,32) mit einem proximalen und einem distalen Ende, einer Öffnung in dem distalen Ende, einem Führungsdrahtlumen (17,26), das sich zu der Öffnung in dem distalen Ende erstreckt, um den Vorschub des Katheters (10) über einen Führungsdraht (18) in dem Gefäßsystem eines Patienten zu erleichtern, und mit einem Inflationslumen (25,16), das sich darin an eine Position erstreckt, die proximal zu dem distalen Ende beabstandet angeordnet ist;
b) einen Ballon (12,34) an einem distalen Teilstück des Katheterschafts (11,32) mit einem Innenraum, der sich in Fluidkommunikation mit dem Inflationslumen (25,16) des Katheterschafts befindet, und der eine elastische Expansionsrate beim Aufblasen in einem ersten Druckbereich über 8 Atmosphären aufweist, wodurch eine wesentliche elastische Expansion bereitgestellt wird, und der eine geringere Expansionsrate und sehr wenig Expansion beim Aufblasen in einem zweiten Druckbereich aufweist, der höher ist als der erste Druckbereich; und
c) ein Mittel zum Leiten von Aufblasfluid durch das Inflationslumen in das Innere des Ballons (12,34).

2. Katheter (10) nach Anspruch 1, ferner **dadurch gekennzeichnet, daß** der Katheterschaft (11) ein inneres röhrenförmiges Element (15) aufweist, wobei sich das den Führungsdraht aufnehmende innere Lumen (17, 26) darin erstreckt, das sich durch das Innere des Ballons (12) erstreckt.

3. Katheter (10) nach Anspruch 2, ferner **dadurch gekennzeichnet, daß** das innere röhrenförmige Element (15) ein Teilstück (22) dessen aufweist, das bei einem Druck kollabierbar ist, der geringer ist als der erste Druckbereich.

4. Katheter (10, 31) nach einem der Ansprüche 1 bis 3, ferner **dadurch gekennzeichnet, daß** der Ballon (12, 34) aus einem strahlenvernetzten Polymerstoff oder einem wärmeschrumpfbaren Polymerstoff hergestellt wird.

5. Katheter (10, 31) nach Anspruch 4, ferner **dadurch gekennzeichnet, daß** es sich bei dem strahlenvernetzten Polymerstoff um ein polyolefinisches Ionomer handelt, das aus der Gruppe ausgewählt wird, die Natrium-, Zink- und Lithiumionomere umfaßt.

6. Katheter (10, 31) nach einem der Ansprüche 1 bis 5, ferner **dadurch gekennzeichnet, daß** der Ballon (12, 34) bei einer Deflation einen elastischen Rücklauf aufweist.

7. Katheter (10, 31) nach einem der Ansprüche 1 bis 6, ferner **dadurch gekennzeichnet, daß** der Ballon (12, 34) auf der Wärmebehandlungstemperatur gefüllt, abgekühlt und danach wärmegeschrumpft wird.

8. Katheter (10, 31) nach einem der vorstehenden Ansprüche, ferner **dadurch gekennzeichnet, daß** die entleerten transversalen Abmessungen des Ballons (12, 34) nicht um mehr als etwa 10% größer sind als die transversale Abmessung des benachbarten Katheterschafts (11, 32).

9. Katheter (10, 31) nach einem der vorstehenden Ansprüche, mit einer Einrichtung zur Erwärmung (23) des Ballons (12, 34), während sich dieser in dem Körper eines Patienten befindet, um den ersten Druckbereich zu senken.

10. Katheter (10) nach einem der Ansprüche 1 bis 9, ferner **dadurch gekennzeichnet, daß** sich der Führungsdraht (18) aus dem distalen Ende des Ballons (12) erstreckt, wobei das distale Ende des Ballons (12) abdichtend um den Führungsdraht (18) angebracht ist.

11. Verfahren zur Herstellung eines Ballons, wobei das Verfahren durch die folgenden Schritte **gekennzeichnet** ist:
a) Extrudieren eines aus einem olefinischen Ionomer auf erhöhter Temperatur gebildeten röhrenförmigen Produkts;
b) Abkühlen des extrudierten röhrenförmigen Produkts nach dessen Extrusion, um darin eine verhältnismäßig amorphe Struktur zu erreichen;
c) Bestrahlen mindestens eines Teilstücks des amorphen röhrenförmigen Produkts, das einen Ballon bildet;
d) Wärmebehandeln des Teilstücks des röhrenförmigen Produkts, das den Ballon bildet, auf einer Temperatur zwischen etwa 50°C oberhalb und etwa 50°C unterhalb der Kristallschmelztemperatur.

12. Verfahren nach Anspruch 11, ferner **gekennzeichnet durch** die folgenden Schritte:
Erweitern des bestrahlten Teilstücks des röhrenförmigen Produkts auf einen ersten äußeren Durchmesser **durch** Injektion von Inflationsfluid in das Innere des bestrahlten Teilstücks auf einer erhöhten Temperatur, um die Ausdehnung des bestrahlten Teilstücks zu bewirken;
Abkühlen des erweiterten Teilstücks des röhrenförmigen Produkts;
Wärmeschrumpfen des erweiterten Teilstücks des röhrenförmigen Produkts auf einen zweiten äußeren Durchmesser, der deutlich kleiner ist als der erste äußere Durchmesser.

13. Verfahren nach Anspruch 11 oder 12, ferner **dadurch gekennzeichnet, daß** das extrudierte röhrenförmige Produkt auf einer Temperatur zwischen etwa 4,4°C (40°F) und etwa 26,6°C (80°F) über einen Zeitraum von etwa 2 bis etwa 6 Stunden vor der Bestrahlung stabilisiert wird.

14. Verfahren nach Anspruch 13, ferner **dadurch gekennzeichnet, daß** das extrudierte röhrenförmige Produkt nach dem Austreten nach dem Extrusionsvorgang in einem Bad bei einer Temperatur von etwa 4°C (40°F) bis etwa 15°C (60°F) abgeschreckt wird.

15. Verfahren nach Anspruch 11, 12, 13 oder 14, ferner **dadurch gekennzeichnet, daß** das extrudierte röhrenförmige Produkt mit einer Gammastrahlung von etwa 50 bis etwa 70 Mrad bestrahlt wird.

16. Verfahren nach Anspruch 11, 12, 13, 14 oder 15, ferner **dadurch gekennzeichnet, daß** das bestrahlte Teilstück des extrudierten röhrenförmigen Produkts einer Temperatur von etwa 107°C (225°F) bis etwa 121°C (250°F) und einem Fülldruck von etwa 345 x 10³ Pa (50 psi) bis etwa 586 x 10³ Pa (85 psi) ausgesetzt wird, um daraus einen gefüllten Ballon (12, 34) zu bilden.

17. Verfahren nach Anspruch 16, ferner **dadurch gekennzeichnet, daß** der gefüllte Ballon (12, 34) abgekühlt und danach über einen Zeitraum von etwa 5 bis etwa 60 Minuten einer Temperatur von etwa 50°C bis etwa 75°C ausgesetzt wird, um den ausgedehnten Ballon (12, 34) von einem ersten Durchmesser auf einen kleineren zweiten Durchmesser wärmezuschrumpfen.

18. Verfahren nach einem der Ansprüche 11 bis 17, wobei der genannte Ballon (12, 34) aus einem oder mehreren oleophilischen Polymeren gebildet wird, die aus der Gruppe ausgewählt werden, die Zink- und Natriumionomere umfaßt.

19. Verfahren nach Anspruch 18, ferner **dadurch gekennzeichnet, daß** der Ballon aus einem olefinischen Ionomer gebildet wird, das aus der Gruppe ausgewählt wird, die olefinische Natrium-, Zink- und Lithium-Ionomere umfaßt.

## Revendications

1. Cathéter allongé (10), **caractérisé en ce que** le cathéter est un cathéter intravasculaire allongé à ballonnet (10, 31) comportant:
a) une tige allongée (11, 32) de cathéter ayant des extrémités proximale et distale, un orifice dans l'extrémité distale, une lumière (17, 26) pour fil de guidage s'étendant jusqu'à l'orifice dans l'extrémité distale pour faciliter l'avance du cathéter (10) sur un fil de guidage (18) à l'intérieur du système vasculaire d'un patient, et une lumière de gonflage (25, 16) s'étendant dans celle-ci jusqu'à un emplacement espacé de façon proximale de l'extrémité distale ;
b) un ballonnet (12, 34) sur une partie distale de la tige (11, 32) du cathéter dont l'intérieur est en communication de fluide avec la lumière de gonflage (25, 16) de la tige (11, 32) du cathéter et présentant un taux d'expansion élastique lors d'un gonflage dans une première plage de pression au-dessus de 8 atmosphères réalisant une expansion élastique substantielle et présentant un taux d'expansion plus faible et très peu d'expansion lors d'un gonflage dans une seconde plage de pression plus élevée que la première plage de pression ; et
c) des moyens destinés à diriger un fluide de gonflage vers l'intérieur du ballonnet (12, 34) en passant par la lumière de gonflage.

2. Cathéter (10) selon la revendication 1, **caractérisé en outre en ce que** la tige (11) du cathéter comporte un élément tubulaire intérieur (15), dans lequel s'étend la lumière intérieure (17, 26) de réception d'un fil de guidage, qui s'étend à travers l'intérieur du ballonnet (12).

3. Cathéter (10) selon la revendication 2, **caractérisé en outre en ce qu'**une partie (22) de l'élément tubulaire intérieur (15) peut s'affaisser à une pression inférieure à la première plage de pression.

4. Cathéter (10, 31) selon l'une quelconque des revendications 1 à 3, **caractérisé en outre en ce que** le ballonnet (12, 34) est formé d'une matière polymérique réticulée par rayonnement ou d'une matière polymérique thermorétractable.

5. Cathéter (10, 31) selon la revendication 4, **caractérisé en outre en ce que** la matière polymérique réticulée par rayonnement est un ionomère polyoléfinique choisi dans le groupe constitué de ionomères de sodium, de zinc et de lithium.

6. Cathéter (10, 31) selon l'une quelconque des revendications 1 à 5, **caractérisé en outre en ce que** le ballonnet (12, 34) présente une détente élastique lors d'un dégonflage.

7. Cathéter (10, 31) selon l'une quelconque des revendications 1 à 6, **caractérisé en outre en ce que** le ballonnet (12, 34) est gonflé à la température d'un traitement thermique, refroidi, puis thermorétracté.

8. Cathéter (10, 31) selon l'une quelconque des revendications précédentes, **caractérisé en outre en ce que** les dimensions transversales du ballonnet (12, 34), dans l'état dégonflé, ne sont pas supérieures de plus d'environ 10% à la dimension transversale de la tige adjacente (11, 32) du cathéter.

9. Cathéter (10, 31) selon l'une quelconque des revendications précédentes, comprenant des moyens pour chauffer (23) le ballonnet (12, 34) alors qu'il est disposé à l'intérieur du corps d'un patient afin d'abaisser la première plage de pression.

10. Cathéter (10) selon l'une quelconque des revendications 1 à 9, **caractérisé en outre en ce que** le fil de guidage (18) sort de l'extrémité distale du ballonnet (12), l'extrémité distale du ballonnet (12) étant fixée de façon scellée autour du fil de guidage (18).

11. Procédé de réalisation d'un ballonnet, **caractérisé par** les étapes dans lesquelles :
a) on extrude le produit tubulaire formé d'un ionomère oléfinique à une température élevée ;
b) on refroidit le produit tubulaire extrudé après son extrusion afin d'obtenir dans ce produit une structure relativement amorphe ;
c) on irradie au moins une partie du produit tubulaire amorphe qui formera un ballonnet ; et
d) on soumet à un traitement thermique la partie du produit tubulaire qui formera le ballonnet, à une température comprise entre environ 50°C au-dessus et environ 50°C au-dessous de la température de fusion cristalline.

12. Procédé selon la revendication 11, **caractérisé en outre par** les étapes dans lesquelles:
on expanse la partie irradiée du produit tubulaire à un premier diamètre extérieur en injectant un fluide de gonflage à l'intérieur de la partie irradiée, à une température élevée, afin de provoquer l'expansion de la partie irradiée ;
on refroidit la partie expansée du produit tubulaire ;
on soumet à un thermoretrait la partie expansée du produit tubulaire jusqu'à un second diamètre extérieur très inférieur au premier diamètre extérieur.

13. Procédé selon la revendication 11 ou 12, **caractérisé en outre en ce que** le produit tubulaire extrudé est stabilisé à une température comprise entre environ 4,4°C (40°F) et environ 26,6°C (80°F) pendant environ 2 à environ 6 heures avant son irradiation.

14. Procédé selon la revendication 13, **caractérisé en outre en ce que** le produit tubulaire extrudé est refroidi rapidement à sa sortie de l'opération d'extrusion dans un bain à une température d'environ 4°C (40°F) à environ 15°C (60°F) .

15. Procédé selon la revendication 11, 12, 13 ou 14, **caractérisé en outre en ce que** le produit tubulaire extrudé est irradié avec environ 50 à environ 70 Mrads d'un rayonnement gamma.

16. Procédé selon la revendication 11, 12, 13, 14 ou 15, **caractérisé en outre en ce que** la partie irradiée du produit tubulaire extrudé est soumise à une température d'environ 107°C (225°F) à environ 121°C (250°F) et à une pression de gonflage d'environ 345 x 10³ Pa (50 psi) à environ 586 x 10³ Pa (85 psi) pour être formée en un ballonnet gonflé (12, 34).

17. Procédé selon la revendication 16, **caractérisé en outre en ce que** le ballonnet gonflé (12, 34) est refroidi puis soumis à une température d'environ 50°C à environ 75°C pendant environ 5 à environ 60 minutes afin que le ballonnet expansé (12, 34) soit thermorétracté d'un premier diamètre à un second diamètre plus petit.

18. Procédé selon l'une quelconque des revendications 11 à 17, dans lequel ledit ballonnet (12, 34) est formé d'un ou plusieurs polymères oléophiliques choisis dans le groupe constitué d'ionomères de zinc et de sodium.

19. Procédé selon la revendication 18, **caractérisé en outre en ce que** le ballonnet est formé d'un ionomère oléfinique choisi dans le groupe constitué d'ionomères oléfiniques de sodium, de zinc et de lithium.
